# EUROPEAN PATENT APPLICATION

(11) **EP 4 725 386 A1**
(43) Date of publication of application: **15.04.2026**
(21) Application number: 24839720.0
(22) Date of filing: 08.07.2024
(51) Int. Cl.: A61B 1/00, A61B 1/005, A61B 1/01, A61B 1/307

(54) **ROBOT SYSTEM AND METHOD FOR CONTROLLING ROBOT SYSTEM**

(30) Priority: 10.07.2023 JP 2023112874
(71) Applicant: KAWASAKI JUKOGYO KABUSHIKI KAISHA, Kobe-shi, Hyogo 650-8670 (JP)
(72) Inventor: KOKUSHI Hiroki, Kobe-Shi, Hyogo 650-8670 (JP); RYU Hideyuki, Kobe-Shi, Hyogo 650-8670 (JP); MIYASHITA Keisuke, Kobe-Shi, Hyogo 650-8670 (JP)
(74) Representative: Hoffmann Eitle
(86) International application number: PCT/JP2024/024632
(87) International publication number: WO 2025/013830

(57) **Abstract**

A robot system (100) includes a controller (30) configured or programmed to store, in a storage (40), information about a movement path (M) of a treatment instrument (200) and control operation of a robot (10) to retrace the movement path (M) of the treatment instrument (200) based on the information about the movement path (M) of the treatment instrument (200) stored in the storage (40).

## Description

### Technical Field

The present disclosure relates to a robot system and a method for controlling a robot system.

### Background Art

Conventionally, robots have been disclosed that hold treatment instruments to be inserted into a patient. Japanese Patent Laid-Open No. 2015-016181 discloses a surgical robot configured to insert an endoscope into the body of a patient. This surgical robot includes an operation unit for operating the endoscope. The operation unit receives operations for bending the endoscope and for moving the endoscope three-dimensionally.

### Prior Art

### Patent Document

Patent Document 1: Japanese Patent Laid-Open No. 2015-016181

### Summary of the Invention

In conventional surgical robots, such as the surgical robot disclosed in Japanese Patent Laid-Open No. 2015-016181, an operation unit may be used to move a treatment instrument such as an endoscope toward a target position along a complex path that may branch and bend. In such a case, after the treatment instrument has reached the target position, the operation unit may be used to retrace the movement path of the treatment instrument back to its original position. In such a case, a user must remember operations performed on the operation unit when the treatment instrument is moved toward the target position, and then perform the operations on the operation unit in reverse order to move the treatment instrument back along the movement path of the treatment instrument. However, this requires the user to remember all of the operations performed to move the treatment instrument, which increases the burden on the user. When the user fails to recall all of the operations performed to move the treatment instrument, the movement path of the treatment instrument may not be accurately retraced.

The present disclosure is intended to solve the above problem. The present disclosure aims to provide a robot system and a method for controlling a robot system each capable of reducing the burden on a user when a treatment instrument is operated to retrace a movement path of the treatment instrument and capable of accurately retracing the movement path of the treatment instrument.

In order to attain the aforementioned object, a robot system according to a first aspect of the present disclosure includes a robot to hold a treatment instrument to be inserted into a patient, an operation unit to receive an operation for the robot, a controller configured or programmed to control operation of the robot based on the operation received by the operation unit, and a storage. The controller is configured or programmed to store, in the storage, information about a movement path of the treatment instrument configured to pass through a body of the patient, and control the operation of the robot to retrace the movement path of the treatment instrument based on the information about the movement path of the treatment instrument stored in the storage.

In the robot system according to the first aspect of the present disclosure, as described above, the controller is configured or programmed to store, in the storage, the information about the movement path of the treatment instrument configured to pass through the body of the patient, and control the operation of the robot to retrace the movement path of the treatment instrument based on the information about the movement path of the treatment instrument stored in the storage. Accordingly, based on the information about the movement path of the treatment instrument stored in the storage, the robot automatically operates to accurately retrace the movement path of the treatment instrument, and thus a user does not need to remember operations required to move the treatment instrument. Consequently, the burden on the user can be reduced when the treatment instrument is operated to retrace the movement path of the treatment instrument, and the movement path of the treatment instrument can be accurately retraced.

In order to attain the aforementioned object, a method for controlling a robot system according to a second aspect of the present disclosure includes storing, in a storage, information about a movement path along which a treatment instrument held by a robot passes through a body of a patient as the robot operates based on an operation received by an operation unit, and controlling operation of the robot to retrace the movement path of the treatment instrument based on the information about the movement path of the treatment instrument stored in the storage.

As described above, the method for controlling a robot system according to the second aspect of the present disclosure includes controlling the operation of the robot to retrace the movement path of the treatment instrument based on the information about the movement path of the treatment instrument stored in the storage. Accordingly, based on the information about the movement path of the treatment instrument stored in the storage, the robot automatically operates to accurately retrace the movement path of the treatment instrument, and thus a user does not need to remember operations required to move the treatment instrument. Consequently, it is possible to provide the method for controlling a robot system capable of reducing the burden on the user when the treatment instrument is operated to retrace the movement path of the treatment instrument and capable of accurately retracing the movement path of the treatment instrument.

As described above, the robot system and the method for controlling the robot system according to the present disclosure can reduce the burden on the user when the treatment instrument is operated to retrace the movement path of the treatment instrument, and can accurately retrace the movement path of the treatment instrument.

### Brief Description of the Drawings

FIG. 1 is a diagram showing a robot system according to a first embodiment.
FIG. 2 is a diagram showing a hand of a robot according to the first embodiment.
FIG. 3 is a block diagram of the robot system according to the first embodiment.
FIG. 4 is a diagram showing a ureteroscope in a bent state.
FIG. 5 is a diagram showing an operation unit according to the first embodiment.
FIG. 6 is a diagram showing the kidney of a patient and a movement path of the ureteroscope.
FIG. 7 is a diagram showing a state in which forward and backward movement paths of the ureteroscope overlap.
FIG. 8 is a diagram for illustrating a state in which the ureteroscope is bent or rotated without moving in a direction A.
FIG. 9 is a flowchart of a method for controlling the robot system according to the first embodiment.
FIG. 10 is a perspective view of a hand according to a second embodiment to which a laser fiber is attached.
FIG. 11 is a perspective view of the hand according to the second embodiment to which a basket forceps is attached.
FIG. 12 is a perspective view of the hand according to the second embodiment.
FIG. 13 is a diagram showing a state in which an engagement claw of the hand according to the second embodiment engages with an engagement claw of a drape adapter.
FIG. 14 is a perspective view of the front side of the drape adapter according to the second embodiment.
FIG. 15 is a perspective view of the back side of the drape adapter according to the second embodiment.
FIG. 16 is a diagram showing the inside of the drape adapter according to the second embodiment.
FIG. 17 is a perspective view of the front side of an endoscope adapter according to the second embodiment.
FIG. 18 is a perspective view of the back side of the endoscope adapter according to the second embodiment.
FIG. 19 is a diagram showing the inside of the endoscope adapter according to the second embodiment.
FIG. 20 is a perspective view of the front side of a laser fiber adapter according to the second embodiment.
FIG. 21 is a perspective view of the back side of the laser fiber adapter according to the second embodiment.
FIG. 22 is a diagram showing the inside of the laser fiber adapter according to the second embodiment.
FIG. 23 is a perspective view of the front side of a basket forceps adapter according to the second embodiment.
FIG. 24 is a perspective view of the back side of the basket forceps adapter according to the second embodiment.
FIG. 25 is a diagram showing the inside of the basket forceps adaptor according to the second embodiment.

### Modes for Carrying Out the Invention

### First Embodiment

A first embodiment embodying the present disclosure is hereinafter described on the basis of the drawings.

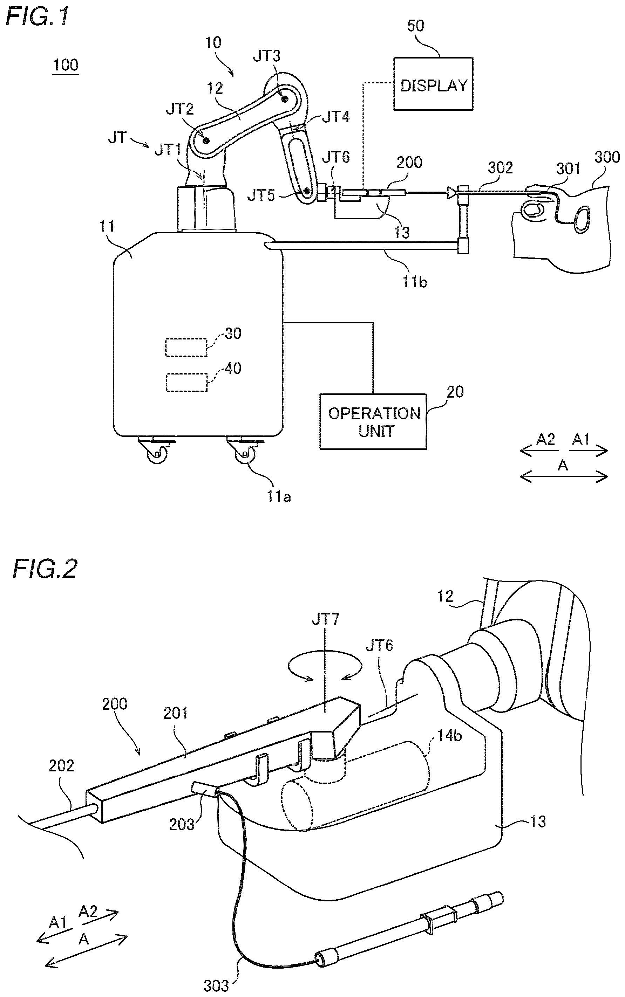

As shown in FIG. 1, a robot system 100 performs a ureteroscopic surgical procedure on a patient 300 using a ureteroscope 200 to fragment urinary stones. The robot system 100 inserts the ureteroscope 200 into the urethra 301 of the patient 300 through a ureteral access sheath 302 that has been inserted into the urethra 301 of the patient 300 in advance by a user, and images the inside of the urinary tract of the patient 300, including the urethra 301 and ureters. The captured image is displayed on a display 50. The user performs the ureteroscopic surgical procedure on the patient 300 by operating a robot 10 using an operation unit 20 while checking a real-time image of the patient 300 displayed on the display 50. In this specification, a direction in which the robot system 100 moves the ureteroscope 200 in and out of the patient 300 is defined as a direction A.

### Configuration of Robot System

As shown in FIG. 1, the robot system 100 includes the robot 10, the operation unit 20, a controller 30, a storage 40, and the display 50. The robot 10 also includes a robot base 11, a robot arm 12, and a hand 13.

The robot arm 12 is placed on the robot base 11. Wheels 11a are disposed on the lower surface of the robot base 11, and the user can manually move the robot base 11. In addition, a support 11b, which supports the ureteral access sheath 302 from below, is attached to the robot base 11.

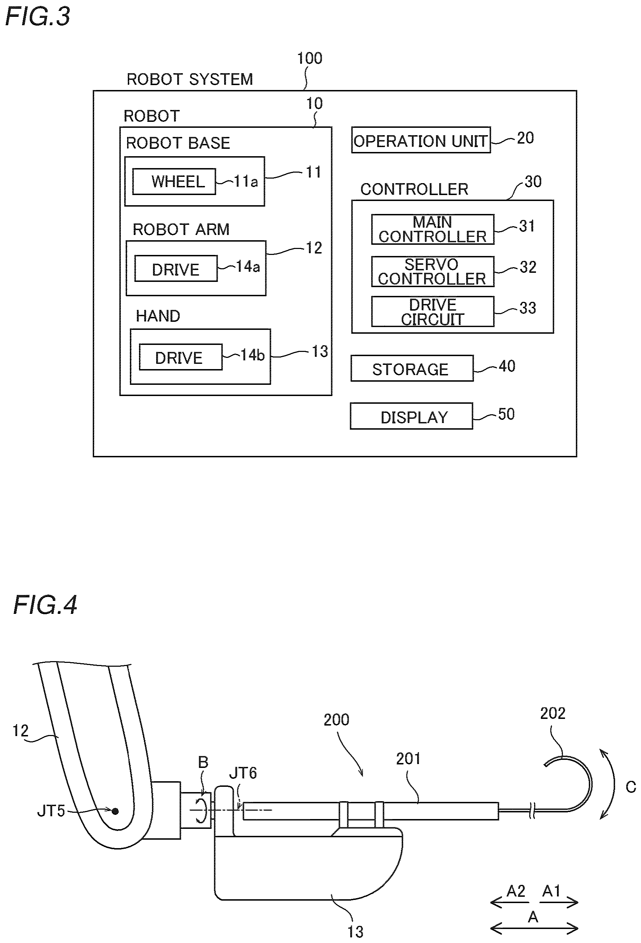

In the first embodiment, the robot arm 12 holds the ureteroscope 200 and is a vertical articulated robot arm 12. The robot arm 12 includes a plurality of joint shafts JT. For example, the robot arm 12 includes six joint shafts JT1, JT2, JT3, JT4, JT5, and JT6. A drive 14a shown in FIG. 3 is disposed at each of the joint shafts JT1 to JT6. The drive 14a includes a servomotor, for example.

The storage 40 includes memories such as a RAM and a ROM, a hard disk, etc. The storage 40 is disposed in the robot base 11, for example.

In the first embodiment, as shown in FIG. 2, an operation shaft JT7 for operating the ureteroscope 200 is disposed in the hand 13. The hand 13 is attached to a distal end of the robot arm 12. A drive 14b is disposed on the operation shaft JT7.

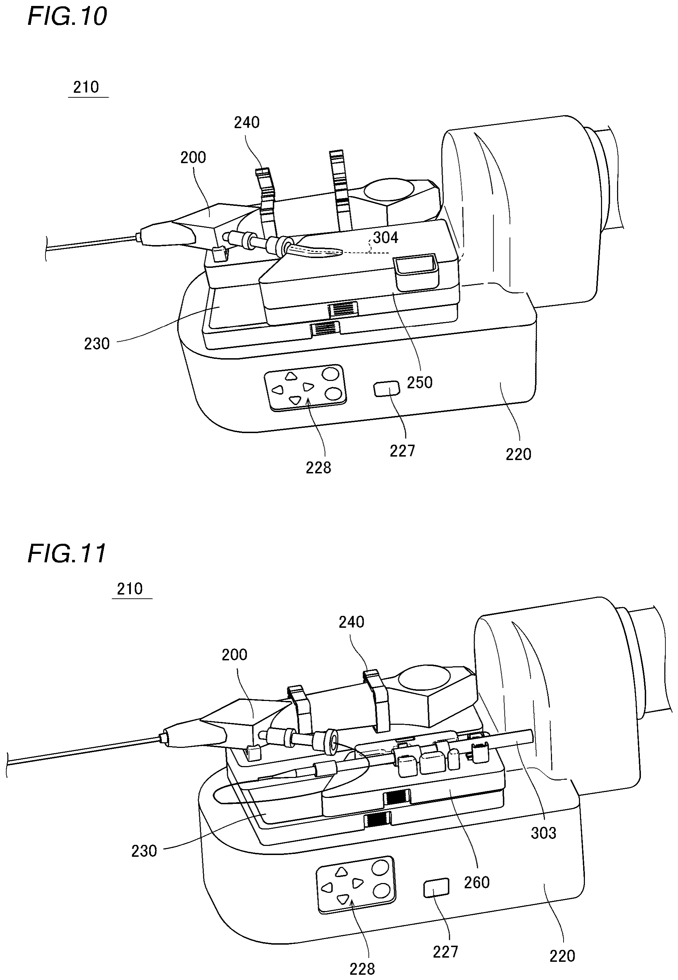

The ureteroscope 200 images the inside of the urinary tract of the patient 300. The ureteroscope 200 includes a main body 201 and an inserted portion 202. The main body 201 is attached to the hand 13. The inserted portion 202 is inserted into the urinary tract of the patient 300. The ureteroscope 200 includes a hole 203 into which a laser fiber 304 shown in FIG. 10 for fragmenting urinary stones in the patient 300 or a basket forceps 303 for retrieving the urinary stones is inserted. The laser fiber 304 or the basket forceps 303 is inserted into the urinary tract of the patient 300 through the hole 203. The ureteroscopic surgical procedure involves identifying the locations of the stones in the patient 300, irradiating the stones with laser light using the laser fiber 304 to fragment the stones, and retrieving the stones using the basket forceps 303, for example.

As shown in FIG. 3, the controller 30 controls the operation of the robot 10 based on an operation received by the operation unit 20. The controller 30 includes a main controller 31, a servo controller 32, and a drive circuit 33. The main controller 31 and the servo controller 32 each include a central processing unit (CPU), for example. The main controller 31 controls the joint shafts JT and the operation shaft JT7 of the robot 10. The servo controller 32 controls power supplied to the drives 14a of the joint shafts JT and the drive 14b of the operation shaft JT7 based on commands from the main controller 31. The controller 30 is a robot controller. The controller 30 is disposed in the robot base 11, for example.

The drive circuit 33 supplies drive power to the drives 14a of the joint shafts JT and the drive 14b of the operation shaft JT7. The drive circuit 33 includes an inverter circuit that supplies AC power as drive power. The drive circuit 33 is provided for each of the joint shafts JT and the operation shaft JT7.

The drive 14a serves as a drive source for operating the robot 10. The drive 14a includes a servomotor, an encoder, and a speed reducer. The servomotor rotates when power is supplied thereto. The servomotor rotates its rotation shaft using three-phase AC power, for example. The encoder detects the rotation angle of the servomotor. The encoder then outputs a detection value indicating the detected rotation angle of the servomotor to the main controller 31 and the servo controller 32. The same applies to the drive 14b.

As shown in FIG. 4, the robot arm 12 moves the ureteroscope 200 in the direction A. The joint shaft JT6 of the robot arm 12 rotates the ureteroscope 200 in a direction B, which is around an axis along the direction A. The drive 14b of the operation shaft JT7 bends the ureteroscope 200 in a direction C, which intersects with the direction A.

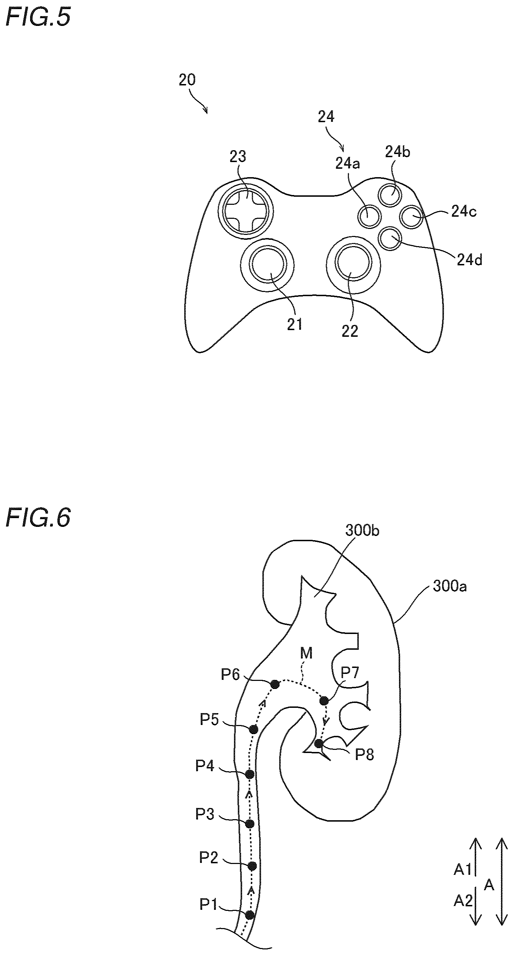

As shown in FIG. 5, the operation unit 20 receives operations for the robot 10. The operation unit 20 includes joysticks 21 and 22, a directional pad 23, and a plurality of buttons 24. For example, the operation unit 20 includes four buttons 24a, 24b, 24c, and 24d. For example, when the joystick 21 is operated in an upward-downward direction on the plane of the figure, the ureteroscope 200 moves in the direction A shown in FIG. 4. When the joystick 22 is operated in a right-left direction on the plane of the figure, the ureteroscope 200 rotates in the direction B shown in FIG. 4. When the joystick 22 is operated in the upward-downward direction on the plane of the figure, the inserted portion 202 of the ureteroscope 200 bends in the direction C shown in FIG. 4.

In the first embodiment, the operation unit 20 also receives operations to move the ureteroscope 200 up and down. For example, the directional pad 23 is operated, the robot arm 12 moves the ureteroscope 200 in the upward-downward direction. Thus, the height position of the ureteroscope 200 can be adjusted to the height position of the ureteral access sheath 302 that has been inserted in advance into the urethra 301 of the patient 300.

In the first embodiment, the operation unit 20 includes the button 24a to receive an operation to start the operation of the robot 10 to retrace a movement path M. The button 24a is one of the plurality of buttons 24 disposed on the operation unit 20.

In the first embodiment, the controller 30 stores information about the movement path M of the ureteroscope 200 through the body of the patient 300 in the storage 40. Then, based on the information about the movement path M of the ureteroscope 200 stored in the storage 40, the controller 30 controls the operation of the robot 10 to retrace the movement path M of the ureteroscope 200. For example, as shown in FIG. 6, the ureteroscope 200 is inserted into the renal pelvis 300b of the kidney 300a through the ureter of the patient 300. The renal pelvis 300b includes complex branching and bending portions.

In the first embodiment, the controller 30 stores, in the storage 40, the movement path M of the ureteroscope 200 along which the ureteroscope 200 is inserted into the patient 300. When the ureteroscope 200 is removed from the patient 300, the controller 30 controls the operation of the robot 10 to retrace the movement path M of the ureteroscope 200 along which the ureteroscope 200 is inserted, based on the information about the ureteroscope 200 stored in the storage 40. For example, the user operates the joystick 21 of the operation unit 20 to operate the robot 10 and insert the ureteroscope 200 into the urethra 301 of the patient 300 via the ureteral access sheath 302. The user also operates the joysticks 21 and 22 of the operation unit 20 to insert the ureteroscope 200 into the renal pelvis 300b of the kidney 300a. The controller 30 stores, in the storage 40, the movement path M of the ureteroscope 200 moving from the urethra 301 into the renal pelvis 300b of the kidney 300a. When the ureteroscope 200 is removed from the patient 300, the controller 30 moves the ureteroscope 200 from the renal pelvis 300b of the kidney 300a to the outside of the urethra 301 based on the information about the ureteroscope 200 stored in the storage 40.

In the first embodiment, the controller 30 executes a process to store, in the storage 40, at least one of the position of the ureteroscope 200 in a forward-backward direction, the rotation angle of the ureteroscope 200, or the bending angle of the ureteroscope 200 at predetermined time intervals or at predetermined movement distance intervals, as the information about the movement path M of the ureteroscope 200. For example, in the first embodiment, the position of the ureteroscope 200 in the forward-backward direction, the rotation angle of the ureteroscope 200, and the bending angle of the ureteroscope 200 are all stored in the storage 40. Furthermore, for example, as shown in FIG. 6, at each of positions P1 to P8 on the movement path M of the ureteroscope 200 at the predetermined time intervals, the position of the ureteroscope 200 in the direction A, which corresponds to the forward-backward direction, the rotation angle of the ureteroscope 200, and the bending angle of the ureteroscope 200 are stored in the storage 40. The position of the ureteroscope 200 in the forward-backward direction can be determined from the robot coordinates. The rotation angle of the ureteroscope 200 can be obtained from the encoder disposed at the joint shaft JT6 of the robot arm 12. The bending angle of the ureteroscope 200 can be obtained from the encoder disposed at the operation shaft JT7. The predetermined time interval is about 0.01 ms, for example. The predetermined movement distance is several µm, for example. The controller 30 controls the operation of the robot 10 to retrace the movement path M of the ureteroscope 200 based on at least one of the position of the ureteroscope 200 in the forward-backward direction, the rotation angle of the ureteroscope 200, or the bending angle of the ureteroscope 200 stored in the storage 40. For example, in the first embodiment, the robot 10 is operated to retrace the movement path M from the position P8 to the position P1 based on the position of the ureteroscope 200 in the forward-backward direction, the rotation angle of the ureteroscope 200, and the bending angle of the ureteroscope 200 at each position from the position P8 to the position P1. For convenience of description, eight positions from the position P1 to the position P8 are shown in FIG. 6, but in reality, information about the movement path M at many positions is stored.

In the first embodiment, the controller 30 controls the operation of the robot arm 12 and the operation of the operation shaft JT7 to retrace the movement path M of the ureteroscope 200, based on the information about the movement path M of the ureteroscope 200 stored in the storage 40. Specifically, the controller 30 executes a process to operate the joint shafts JT1 to JT6 and the operation shaft JT7, based on the information about the movement path M of the ureteroscope 200 stored in the storage 40. That is, the controller 30 executes a process to operate the robot arm 12 and the operation shaft JT7 to retrace the movement path M of the ureteroscope 200 while moving the ureteroscope 200 in the direction A by driving the joint shafts JT1 to JT5, rotating the ureteroscope 200 in the direction B by driving the joint shaft JT6, and bending the ureteroscope 200 in the direction C by driving the operation shaft JT7.

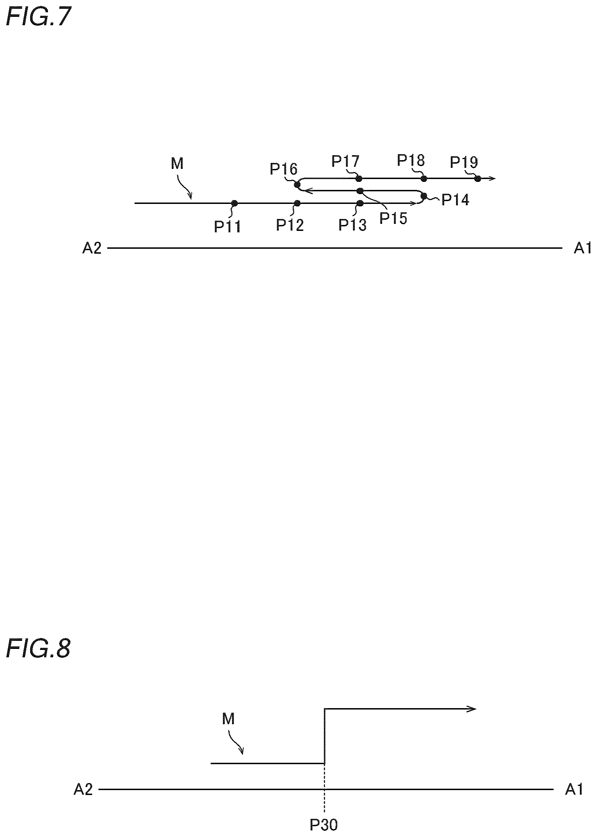

In the first embodiment, when the ureteroscope 200 moves back and forth within the body of the patient 300 and the forward and backward paths of the ureteroscope 200 overlap, the controller 30 executes a process to delete at least a portion of an overlapping portion of the information about the movement path M previously stored in the storage 40. For example, as shown in FIG. 7, assume that the ureteroscope 200 moves along the direction A toward the A1 side via a position P11, turns back toward the A2 side at P14, and then turns back again toward the A1 side at P16. In such a case, the movement path M from positions P12 to P14, the movement path M from positions P14 to P16, and the movement path M from positions P16 to P18 overlap each other in the direction A, and thus the controller 30 deletes these movement paths M from the storage 40. That is, the controller 30 deletes the movement path M from the positions P12 to P14 and the movement path M from the positions P14 to P16, and stores positions P11, P16, P17, P18, and P19 in the storage 40 as the movement path M. That is, in the first embodiment, the entire overlapping portion of the information about the movement path M is deleted from the storage 40.

In the first embodiment, the user presses the button 24a shown in FIG. 5 to start the operation of the robot 10 in order for the ureteroscope 200 to retrace the movement path M. Specifically, when the ureteroscope 200 is removed from the patient 300, the user presses the button 24a to start the operation of the robot 10 to retrace the movement path M of the ureteroscope 200 along which the ureteroscope 200 is inserted. The button 24a is an example of a first operation unit.

In the first embodiment, while the button 24a is continuously operated, the robot 10 retraces the movement path M of the ureteroscope 200. In other words, when the user continues to press the button 24a, the robot 10 operates such that the ureteroscope 200 retraces the movement path M from the position P8 toward the position P1. When the user releases the button 24a, movement of the ureteroscope 200 is stopped.

In the first embodiment, the controller 30 controls the operation of the robot 10 such that the ureteroscope 200 moves at a predetermined speed or less when the movement path M of the ureteroscope 200 is retraced. For example, the predetermined speed is equal to or less than the maximum speed at which the ureteroscope 200 moves in the direction A when the joystick 21 of the operation unit 20 is operated. As shown in FIG. 8, for example, assume that the ureteroscope 200 is rotated or bent at a position P30 while movement of the ureteroscope 200 in the direction A is stopped. In such a case, when the ureteroscope 200 is moved at the position P30 at the same speed as before and after the position P30 while the movement path M of the ureteroscope 200 is retraced from the A1 side to the A2 side, the ureteroscope 200 is rotated or bent rapidly at the position P30. Therefore, the controller 30 executes a process to reduce the movement speed of the ureteroscope 200 in the vicinity of the position P30 so as to prevent the ureteroscope 200 from being rotated or bent rapidly at the position P30 while the movement path M of the ureteroscope 200 is retraced.

### Method for Controlling Robot System

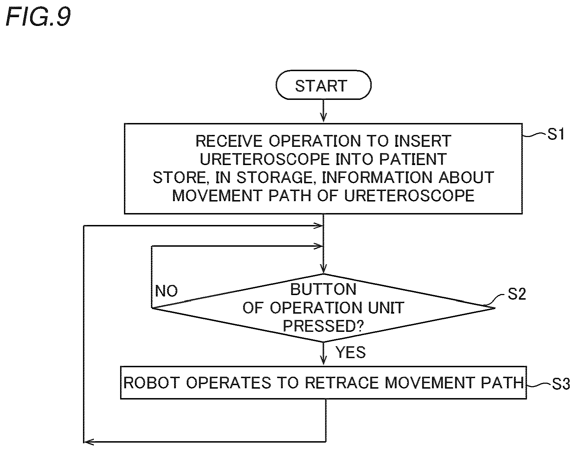

A method for controlling the robot system 100 is now described with reference to FIG. 9.

In step S1, the operation unit 20 receives an operation to insert the ureteroscope 200 into the patient 300. At this time, the controller 30 executes a process to store, in the storage 40, the information about the movement path M of the ureteroscope 200 along which the ureteroscope 200 is inserted into the patient 300.

In step S2, the controller 30 determines whether or not the button 24a of the operation unit 20 has been pressed.

When the result is yes in step S2, then in step S3, controller 30 controls the operation of the robot 10 to retrace the movement path M of the ureteroscope 200 along which the ureteroscope 200 is inserted, based on the information about the ureteroscope 200 stored in the storage 40. The operations in step S2 and step S3 are repeated while the button 24a of the operation unit 20 is pressed. When the result is no in step S2, the determination of step S2 is repeated. Furthermore, the operations in step S2 and step S3 are repeated until the ureteroscope 200 is removed from the patient 300.

### Advantageous Effects of First Embodiment

The controller 30 is configured or programmed to store, in the storage 40, the information about the movement path M of the ureteroscope 200 configured to pass through the body of the patient 300, and control the operation of the robot 10 to retrace the movement path M of the ureteroscope 200 based on the information about the movement path M of the ureteroscope 200 stored in the storage 40. Accordingly, based on the information about the movement path M of the ureteroscope 200 stored in the storage 40, the robot 10 automatically operates to accurately retrace the movement path M of the ureteroscope 200, and thus the user does not need to remember operations required to move the ureteroscope 200. Consequently, the burden on the user can be reduced when the ureteroscope 200 is operated to retrace the movement path M of the ureteroscope 200, and the movement path M of the ureteroscope 200 can be accurately retraced.

It is also conceivable that the patient 300 is imaged using an X-ray apparatus, and the user operates the operation unit 20 to retrace the movement path M of the ureteroscope 200 while visually recognizing the captured X-ray image. However, the X-ray image is a two-dimensional image, and thus it is difficult for the user to accurately understand the three-dimensional movement path M. Furthermore, X-ray imaging exposes the patient 300 and the user to radiation. Therefore, the controller 30 executes a process to operate the robot 10 to retrace the movement path M of the ureteroscope 200 based on the information about the movement path M of the ureteroscope 200 stored in the storage 40 such that the movement path M of the ureteroscope 200 can be accurately retraced while radiation exposure to the patient 300 and the user is reduced or prevented.

The controller 30 is configured or programmed to store, in the storage 40, the information about the movement path M of the ureteroscope 200 along which the ureteroscope 200 is inserted into the patient 300, and control the operation of the robot 10 to retrace the movement path M of the ureteroscope 200 along which the ureteroscope 200 is inserted based on the information about the ureteroscope 200 stored in the storage 40 when the ureteroscope 200 is removed from the patient 300. Accordingly, when the ureteroscope 200 is removed from the patient 300, the robot 10 automatically operates to retrace the movement path M of the ureteroscope 200, and thus the burden on the user can be reduced when the ureteroscope 200 is removed from the patient 300.

The operation unit 20 is configured to receive operations to rotate and bend the ureteroscope 200 and to move the ureteroscope 200 in the forward-backward direction. Accordingly, when the ureteroscope 200 is rotated and/or bent to retrace the movement path M of the ureteroscope 200, or when the ureteroscope 200 is moved in the forward-backward direction to retrace the movement path M of the ureteroscope 200, the burden on the user can be reduced.

The controller 30 is configured or programmed to store, in the storage 40, at least one of the position of the ureteroscope 200 in the forward-backward direction, the rotation angle of the ureteroscope 200, or the bending angle of the ureteroscope 200 at the predetermined time intervals or at the predetermined movement distance intervals as the information about the movement path M of the ureteroscope 200, and control the operation of the robot 10 to retrace the movement path M of the ureteroscope 200 based on at least one of the position of the ureteroscope 200 in the forward-backward direction, the rotation angle of the ureteroscope 200, or the bending angle of the ureteroscope 200 stored in the storage 40. Accordingly, the position of the ureteroscope 200 in the forward-backward direction, the rotation angle of the ureteroscope 200, and the bending angle of the ureteroscope 200 can be stored in the storage 40 at relatively short time intervals or at relatively short movement distance intervals, and thus the robot 10 can be operated to accurately retrace the movement path M of the ureteroscope 200.

The operation unit 20 includes the button 24a to receive an operation to start the operation of the robot 10 to retrace the movement path M. Accordingly, the user can easily operate the robot 10 to retrace the movement path M of the ureteroscope 200 simply by operating the button 24a.

The robot 10 is configured to retrace the movement path M of the ureteroscope 200 while the button 24a is continuously operated. Accordingly, the user can easily stop the operation of the robot 10 by releasing the operation on the button 24a.

The controller 30 is configured or programmed to control the operation of the robot 10 such that the ureteroscope 200 moves at the predetermined speed or less when the movement path M of the ureteroscope 200 is retraced. Accordingly, movement of the ureteroscope 200 at an excessively high speed can be reduced or prevented.

The controller 30 is configured or programmed to execute a process to delete, from the storage 40, at least a portion of the overlapping portion of the information about the movement path M previously stored in the storage 40 when the ureteroscope 200 moves back and forth within the body of the patient 300 and the forward and backward paths of the ureteroscope 200 overlap. Accordingly, unnecessary forward and backward movement of the ureteroscope 200 is reduced or prevented when the movement path M of the ureteroscope 200 is retraced, and thus the time required for the ureteroscope 200 to move can be reduced.

The robot 10 includes the vertical articulated robot arm 12 to hold the ureteroscope 200. The controller 30 is configured or programmed to control the operation of the robot arm 12 and the operation of the operation shaft JT7 configured to operate the ureteroscope 200 so as to retrace the movement path M of the ureteroscope 200 based on the information about the movement path M of the ureteroscope 200 stored in the storage 40. Accordingly, the burden on the user can be reduced when the ureteroscope 200 is operated to retrace the movement path M of the ureteroscope 200 using the robot arm 12 and the operation shaft JT7.

The operation unit 20 is configured to receive operations to move the ureteroscope 200 up and down. Accordingly, the height position of the ureteroscope 200 can be adjusted to an appropriate height position when the ureteroscope 200 is inserted into the patient 300.

### Second Embodiment

A robot system 210 according to a second embodiment is now described. As shown in FIGS. 10 and 11, in the robot system 210, a ureteroscope 200, a basket forceps 303, and a laser fiber 304 are attached to a hand 220 via adapters. The adapters include a drape adapter 230, an endoscope adapter 240, a laser fiber adapter 250, and a basket forceps adapter 260.

### Hand

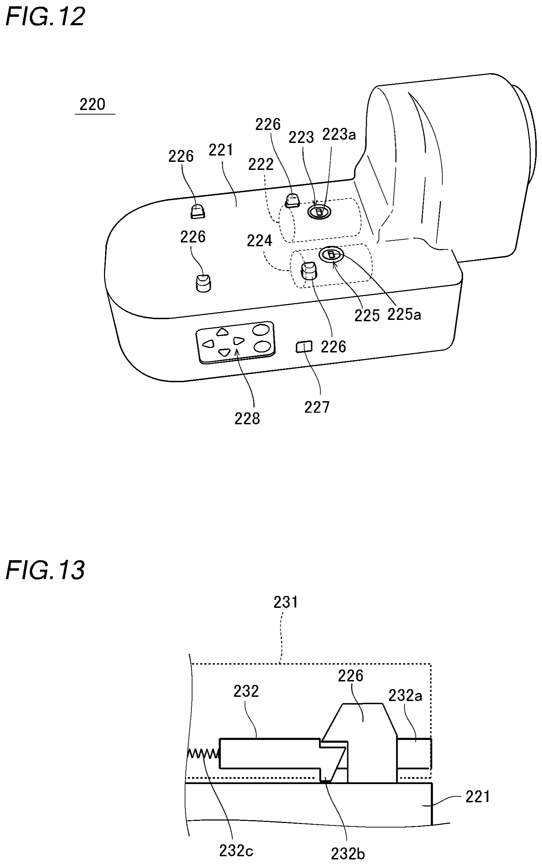

The configuration of the hand 220 is now described. As shown in FIG. 12, the hand 220 includes a housing 221, an endoscope drive 222, a hand rotator 223, a forceps drive 224, a hand rotator 225, and engagement claws 226. The endoscope drive 222 includes a servomotor and a speed reducer. The forceps drive 224 includes a servomotor and a speed reducer. The endoscope drive 222 and the forceps drive 224 are disposed inside the housing 221 of the hand 220. The hand rotator 223 is connected to the speed reducer of the endoscope drive 222 and rotated by the servomotor. The hand rotator 223 is rotatably disposed on a surface of the hand 220. The hand rotator 223 has a disk shape, for example, and includes an engagement recess 223a that engages with a drape adapter rotator 234. The hand rotator 225 is connected to the speed reducer of the forceps drive 224 and rotated by the servomotor. The hand rotator 225 is rotatably disposed on a surface of the housing 221. The hand rotator 225 has a disk shape, for example, and includes an engagement recess 225a that engages with a drape adapter rotator 235. A plurality of engagement claws 226 are disposed, for example. As shown in FIG. 13, the engagement claws 226 engage with engagement claws 232b of the drape adapter 230. The engagement claws 226 each have a convex shape that protrudes from the surface of the housing 221. Distal ends of the engagement claws 226 are chamfered and have hook shapes that engage with the engagement claws 232b.

As shown in FIG. 12, an enable button 227 and jog operation buttons 228 are disposed on the housing 221. The enable button 227 and the jog operation buttons 228 are disposed on each of both sides of the housing 221. The jog operation buttons 228 receive operations to move the hand 220 in an upward-downward direction, to move the hand 220 in a forward-backward direction, and to rotate the hand 220, for example. The above operations are received by simultaneously pressing the enable button 227 and the jog operation buttons 228. For example, when the robot system 210 is moved to the vicinity of a patient 300, a user operates the enable button 227 and the jog operation buttons 228 to fine-tune the position of the hand 220. The user also operates the enable button 227 and the jog operation buttons 228 when performing an operation to move the ureteroscope 200 in a direction in which the ureteroscope 200 is withdrawn from the patient 300 and then reinsert the ureteroscope 200 into the patient 300.

### Drape Adapter

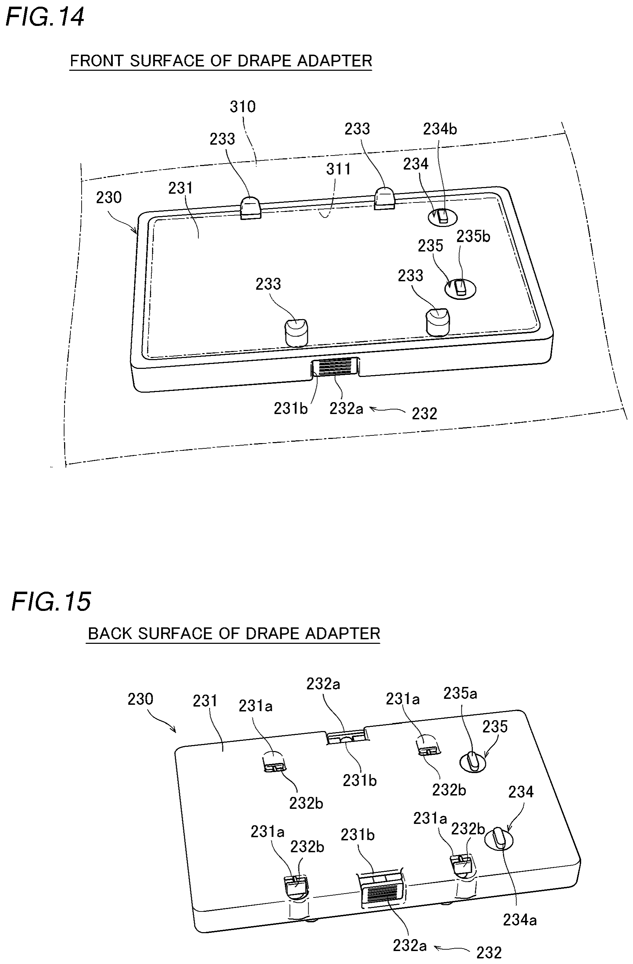

The configuration of the drape adapter 230 is now described. In the second embodiment, as shown in FIG. 14, a drape 310 is attached to the front side of the drape adapter 230. The drape adapter 230 is detachably attached to the hand 220. The endoscope adapter 240 is attached to the hand 220 via the drape adapter 230. The laser fiber adapter 250 and the basket forceps adapter 260 are also attached to the hand 220 via the drape adapter 230. Specifically, the drape adapter 230 includes a housing 231, drape adapter attachment/detachment portions 232, engagement claws 233, the drape adapter rotator 234, and the drape adapter rotator 235. The drape 310 is attached to the front surface of the housing 231 facing the endoscope adapter 240. An opening 311 is formed in the drape 310. The housing 231 is rectangular, and the opening 311 is also rectangular, for example. The drape 310 is attached such that the outer edge of the opening 311 is aligned with the outer edge of the housing 231. Thus, the engagement claws 233 and the drape adapter rotators 234 and 235 of the drape adapter 230 are exposed through the opening 311. The drape 310 is attached to the drape adapter 230 by welding, for example. In such a case, the drape 310 and the drape adapter 230 are integral and unitary with each other, but the drape 310 and the drape adapter 230 may be separable from each other.

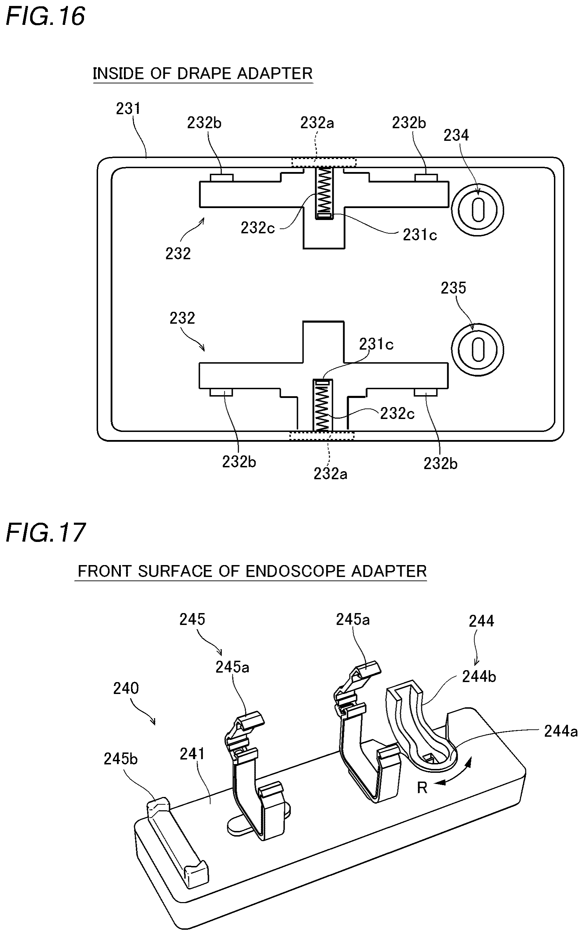

The drape adapter 230 is manually attached to and detached from the hand 220. In the second embodiment, as shown in FIG. 16, the drape adapter attachment/detachment portions 232 receive manual operations to attach and detach the drape adapter 230 to and from the hand 220. A pair of drape adapter attachment/detachment portions 232 are disposed. Each of the pair of drape adapter attachment/detachment portions 232 includes a pressing portion 232a, engagement claws 232b, and a spring 232c. The pressing portion 232a and the engagement claws 232b are integrally formed. The pressing portion 232a has a plate shape that slides inside the housing 231. Furthermore, a first end of the spring 232c contacts a ribbed wall 231c of the housing 231, and a second end of the spring 232c contacts the pressing portion 232a. As shown in FIG. 15, openings 231a are formed in the back surface of the housing 231 facing the hand 220, and the engagement claws 232b are exposed through the openings 231a. When the user presses the drape adapter 230 against the hand 220, the engagement claws 232b of the drape adapter 230 contact the engagement claws 226 of the hand 220, the pressing portion 232a slides, and the engagement claws 226 of the hand 220 enter through the openings 231a of the drape adapter 230. The engagement claws 226 of the hand 220 then engage with the engagement claws 232b of the drape adapter 230. The biasing force of the spring 232c maintains the engagement between the engagement claws 226 of the hand 220 and the engagement claws 232b of the drape adapter 230. Thus, the drape adapter 230 is attached to the hand 220. An opening 231b is formed in each of the side surfaces of the housing 231, and a portion of the pressing portion 232a is exposed through the opening 231b. When the drape adapter 230 is removed from the hand 220, the user presses the portion of the pressing portion 232a exposed through the opening 231b to slide the pressing portion 232a. Thus, the engagement between the engagement claws 226 of the hand 220 and the engagement claws 232b of the drape adapter 230 is released, and the drape adapter 230 can be removed from the hand 220.

In the second embodiment, the drape adapter rotator 234 of the drape adapter 230 is rotated by the driving force of the endoscope drive 222. The drape adapter rotator 235 is rotated by the driving force of the forceps drive 224. The drape adapter rotator 234 and the drape adapter rotator 235 each have a disk shape, for example. The drape adapter rotator 234 and the drape adapter rotator 235 are rotatably attached so as to pass through the drape adapter 230. As shown in FIG. 15, an engagement protrusion 234a that engages with the engagement recess 223a of the hand rotator 223 is formed on the back side of the drape adapter rotator 234. An engagement protrusion 235a that engages with the engagement recess 225a of the hand rotator 225 is formed on the back side of the drape adapter rotator 235. As shown in FIG. 14, an engagement protrusion 234b that engages with an engagement recess 243a of an endoscope adapter rotator 243 is formed on the front side of the drape adapter rotator 234. An engagement protrusion 235b that engages with an engagement recess 253a of a laser fiber adapter rotator 253 or an engagement recess 263a of a basket forceps adapter rotator 263 is formed on the front side of the drape adapter rotator 235.

As shown in FIG. 14, a plurality of engagement claws 233 are disposed on the front side of the drape adapter 230, for example, and engage with engagement claws 242b of the endoscope adapter 240, engagement claws 252b of the laser fiber adapter 250, and engagement claws 262b of the basket forceps adapter 260. The engagement claws 233 each have a convex shape that protrudes from the front surface of the housing 231. Similarly to the engagement claws 226 of the hand 220 shown in FIG. 13, distal ends of the engagement claws 233 of the drape adapter 230 are chamfered and have hook shapes that engage with the engagement claws 242b of the endoscope adapter 240, the engagement claws 252b of the laser fiber adapter 250, and the engagement claws 262b of the basket forceps adapter 260.

### Endoscope Adapter

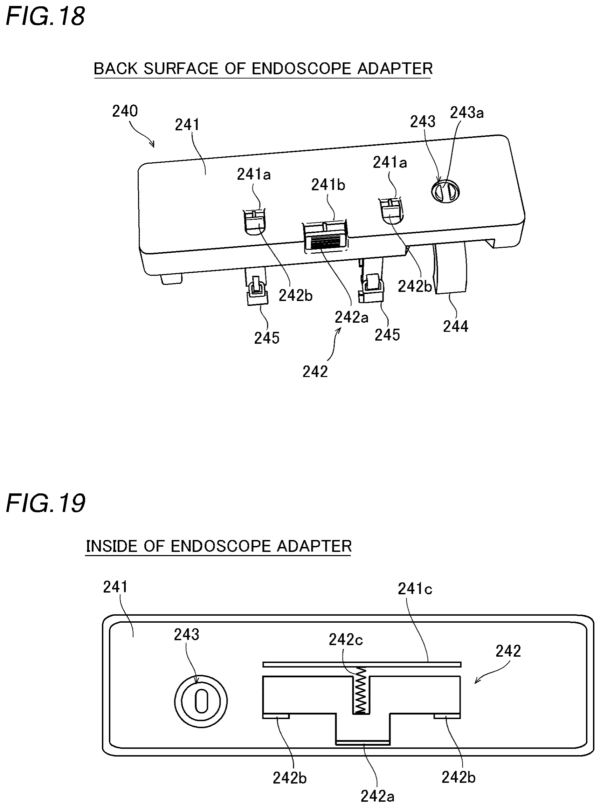

The endoscope adapter 240 is now described. As shown in FIGS. 10 and 11, the endoscope adapter 240 is detachably attached to the hand 220. Specifically, the endoscope adapter 240 is attached to the hand 220 via the drape adapter 230. As shown in FIGS. 17 and 18, the endoscope adapter 240 includes a housing 241, an endoscope adapter attachment/detachment portion 242, the endoscope adapter rotator 243, an endoscope mover 244, and a holder 245.

The endoscope adapter 240 is manually attached to and detached from the hand 220. In the second embodiment, as shown in FIG. 19, the endoscope adapter 240 includes the endoscope adapter attachment/detachment portion 242 that receives manual operations to attach and detach the endoscope adapter 240 to and from the hand 220. The endoscope adapter attachment/detachment portion 242 includes a pressing portion 242a, the engagement claws 242b, and a spring 242c. The pressing portion 242a and the engagement claws 242b are integrally formed. The pressing portion 242a has a plate shape that slides inside the housing 241. A first end of the spring 242c contacts a ribbed wall 241c of the housing 241, and a second end of the spring 242c contacts the pressing portion 242a. As shown in FIG. 18, openings 241a are formed in the back surface of the housing 241 facing the drape adapter 230, and the engagement claws 242b are exposed through the openings 241a. When the user presses the endoscope adapter 240 against the drape adapter 230, the engagement claws 242b of the endoscope adapter 240 contact the engagement claws 233 of the drape adapter 230, the pressing portion 242a slides, and the engagement claws 233 of the drape adapter 230 enter through the openings 241a of the endoscope adapter 240. Thus, the engagement claws 233 of the drape adapter 230 engage with the engagement claws 242b of the endoscope adapter 240. Furthermore, the biasing force of the spring 242c maintains the engagement between the engagement claws 233 of the drape adapter 230 and the engagement claws 242b of the endoscope adapter 240. Thus, the endoscope adapter 240 is attached to the drape adapter 230. An opening 241b is formed in the side surface of the housing 241, and a portion of the pressing portion 242a is exposed through the opening 241b. When the endoscope adapter 240 is removed from the drape adapter 230, the user presses a portion of the pressing portion 242a exposed through the opening 241b to slide the pressing portion 242a. Thus, the engagement between the engagement claws 233 of the drape adapter 230 and the engagement claws 242b of the endoscope adapter 240 is released, and the endoscope adapter 240 can be removed from the drape adapter 230. The engagement between the engagement claws 233 of the drape adapter 230 and the engagement claws 242b of the endoscope adapter 240 is similar to the engagement between the engagement claws 226 of the hand 220 and the engagement claws 232b of the drape adapter 230 shown in FIG. 13.

In the second embodiment, the endoscope adapter rotator 243 engages with the drape adapter rotator 234 and rotates together with the drape adapter rotator 234. The endoscope adapter rotator 243 has a disk shape, for example. As shown in FIG. 18, the engagement recess 243a that engages with the engagement protrusion 234b formed on the front side of the drape adapter rotator 234 is formed on the back surface of the endoscope adapter rotator 243 facing the drape adapter 230. The endoscope mover 244 is attached to the front surface of the endoscope adapter rotator 243 opposite to the drape adapter 230.

As shown in FIG. 17, the endoscope mover 244 is disposed to cover an endoscope operation unit 204 of the ureteroscope 200 shown in FIG. 2. The endoscope mover 244 has an L-shape, for example. A bottom portion 244a of the L-shaped endoscope mover 244 is connected to the endoscope adapter rotator 243. A protrusion 244b protruding from the bottom portion 244a of the endoscope mover 244 has a U-shaped cross-section and covers the endoscope operation unit 204. As the endoscope mover 244 rotates in a direction R due to the driving force of the endoscope drive 61, the endoscope operation unit 204 also rotates in the direction R, and an inserted portion 202 of the ureteroscope 200 is bent.

In the second embodiment, the holder 245 is disposed on the endoscope adapter 240 and holds the ureteroscope 200. Specifically, the holder 245 includes circumferential holding portions 245a that circumferentially surround and hold the ureteroscope 200, and a lower support 245b that supports the ureteroscope 200 from below. For example, two circumferential holding portions 245a are disposed. The circumferential holding portions 245a open and close. With the circumferential holding portions 245a open, the ureteroscope 200 is placed inside the circumferential holding portions 245a and on the lower support 245b, and the ureteroscope 200 is held by closing the circumferential holding portions 245a.

### Laser Fiber Adapter

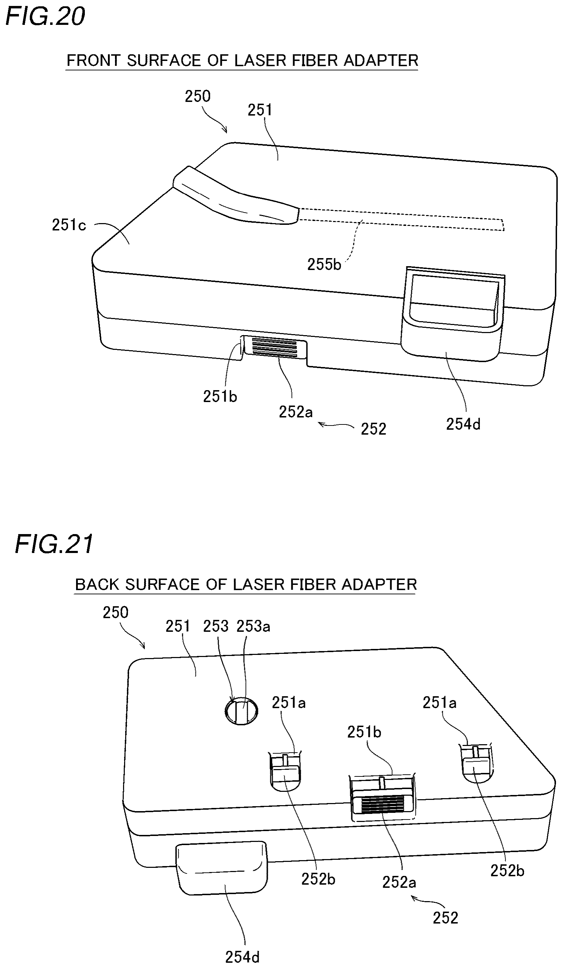

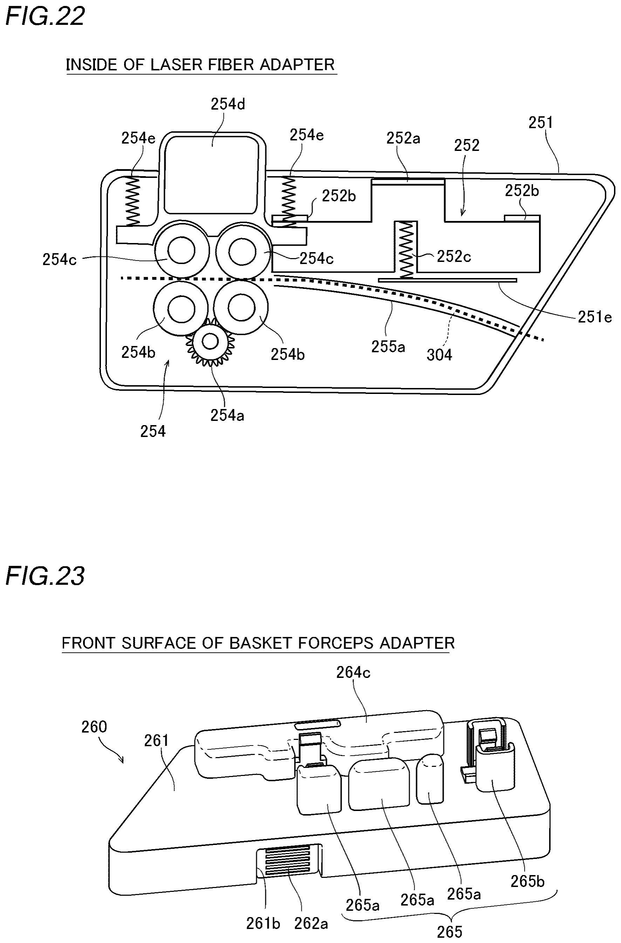

The configuration of the laser fiber adapter 250 is now described. In the second embodiment, as shown in FIG. 10, the laser fiber adapter 250 is detachably attached to the hand 220. Specifically, the laser fiber adapter 250 is attached to the hand 220 via the drape adapter 230. In the second embodiment, either the laser fiber adapter 250 or the basket forceps adapter 260 is attached to the hand 220 via the drape adapter 230. As shown in FIGS. 20 and 21, the laser fiber adapter 250 includes a housing 251, a laser fiber adapter attachment/detachment portion 252, the laser fiber adapter rotator 253, and a laser fiber mover 254 shown in FIG. 22.

The laser fiber adapter 250 is manually attached to and detached from the hand 220. As shown in FIG. 22, the laser fiber adapter 250 includes the laser fiber adapter attachment/detachment portion 252 that receives manual operations to attach and detach the laser fiber adapter 250 to and from the hand 220. The laser fiber adapter attachment/detachment portion 252 includes a pressing portion 252a, the engagement claws 252b, and a spring 252c. The pressing portion 252a and the engagement claws 252b are integrally formed. The pressing portion 252a has a plate shape that slides inside the housing 251. A first end of the spring 252c contacts a ribbed wall 251e of the housing 251, and a second end of the spring 252c contacts the pressing portion 252a. As shown in FIG. 21, openings 251a are formed in the back surface of the housing 251 facing the drape adapter 230, and the engagement claws 252b are exposed through the openings 251a. When the user presses the laser fiber adapter 250 against the drape adapter 230, the engagement claws 252b of the laser fiber adapter 250 contact the engagement claws 233 of the drape adapter 230, the pressing portion 252a slides, and the engagement claws 233 of the drape adapter 230 enter through the openings 251a of the laser fiber adapter 250. Thus, the engagement claws 233 of the drape adapter 230 engage with the engagement claws 252b of the laser fiber adapter 250. Furthermore, the biasing force of the spring 252c maintains the engagement between the engagement claws 233 of the drape adapter 230 and the engagement claws 252b of the laser fiber adapter 250. Thus, the laser fiber adapter 250 is attached to the drape adapter 230. An opening 251b is formed in the side surface of the housing 251, and a portion of the pressing portion 252a is exposed through the opening 251b. When the laser fiber adapter 250 is removed from the drape adapter 230, the user presses the portion of the pressing portion 252a exposed through the opening 251b to slide the pressing portion 252a. Thus, the engagement between the engagement claws 233 of the drape adapter 230 and the engagement claws 252b of the laser fiber adapter 250 is released, and the laser fiber adapter 250 can be removed from the drape adapter 230. The engagement between the engagement claws 233 of the drape adapter 230 and the engagement claws 252b of the laser fiber adapter 250 is similar to the engagement between the engagement claws 226 of the hand 220 and the engagement claws 232b of the drape adapter 230 shown in FIG. 13.

In the second embodiment, the laser fiber adapter rotator 253 engages with the drape adapter rotator 235 and rotates together with the drape adapter rotator 235. The laser fiber adapter rotator 253 has a disk shape, for example. As shown in FIG. 21, the engagement recess 253a that engages with the engagement protrusion 235b of the drape adapter rotator 235 is formed on the back surface of the laser fiber adapter rotator 253 facing the drape adapter 230. As shown in FIG. 22, a laser fiber mover 254 is attached to the front surface of the laser fiber adapter rotator 253 opposite to the drape adapter 230.

As shown in FIG. 22, the laser fiber mover 254 includes a gear 254a and two drive rollers 254b that mesh with the gear 254a. The gear 254a rotates together with the laser fiber adapter rotator 253. The two drive rollers 254b rotate together with the gear 254a. Two driven rollers 254c are disposed to face the two drive rollers 254b. The two driven rollers 254c are attached to a driven roller mover 254d. Springs 254e are also disposed to bias the driven roller mover 254d. In a state in which the user pulls the driven roller mover 254d such that the two driven rollers 254c are spaced apart from the two drive rollers 254b, the user places the laser fiber 304 between the two driven rollers 254c and the two drive rollers 254b. When the user releases the tension on the driven roller mover 254d, the laser fiber 304 is sandwiched between the two driven rollers 254c and the two drive rollers 254b by the biasing force of the springs 254e. Rotation of the two drive rollers 254b moves the laser fiber 304 in and out of the urethra 301 of the patient 300.

As shown in FIG. 22, a groove 255a for guiding the laser fiber 304 is formed inside the housing 251. In addition, as shown in FIG. 20, a groove 255b for guiding the laser fiber 304 is formed inside the upper surface 251c of the housing 251. The laser fiber 304 is led out of the laser fiber adapter 250 from the drive rollers 254b and the driven roller mover 254d via the grooves 255a and 255b.

### Basket Forceps Adapter

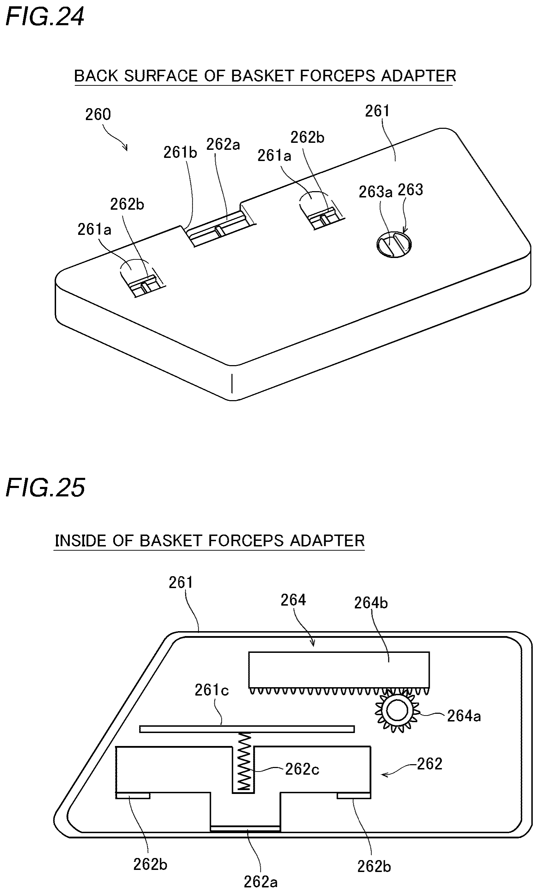

The configuration of the basket forceps adapter 260 is now described. In the second embodiment, as shown in FIG. 11, the basket forceps adapter 260 is detachably attached to the hand 220. Specifically, the basket forceps adapter 260 is attached to the hand 220 via the drape adapter 230. As shown in FIGS. 23 and 24, the basket forceps adapter 260 includes a housing 261, a basket forceps adapter attachment/detachment portion 262, the basket forceps adapter rotator 263, a basket forceps mover 264 shown in FIG. 25, and a holder 265.

The basket forceps adapter 260 is manually attached to and detached from the hand 220. As shown in FIG. 25, the basket forceps adapter 260 includes the basket forceps adapter attachment/detachment portion 262 that receives manual operations to attach and detach the basket forceps adapter 260 to and from the hand 220. The basket forceps adapter attachment/detachment portion 262 includes a pressing portion 262a, the engagement claws 262b, and a spring 262c. The pressing portion 262a and the engagement claws 262b are integrally formed. The pressing portion 262a has a plate shape that slides inside the housing 261. Furthermore, a first end of the spring 262c contacts a ribbed wall 261c of the housing 261, and a second end of the spring 262c contacts the pressing portion 262a. As shown in FIG. 24, openings 261a are formed in the back surface of the housing 261 facing the drape adapter 230, and the engagement claws 262b are exposed through the openings 261a. When the user presses the basket forceps adapter 260 against the drape adapter 230, the engagement claws 262b of the basket forceps adapter 260 contact the engagement claws 233 of the drape adapter 230, the pressing portion 262a slides, and the engagement claws 233 of the drape adapter 230 enter through the openings 261a of the basket forceps adapter 260. Thus, the engagement claws 233 of the drape adapter 230 engage with the engagement claws 262b of the basket forceps adapter 260. Furthermore, the biasing force of the spring 262c maintains the engagement between the engagement claws 233 of the drape adapter 230 and the engagement claws 262b of the basket forceps adapter 260. Thus, the basket forceps adapter 260 is attached to the drape adapter 230. An opening 261b is formed in the side surface of the housing 261, and a portion of the pressing portion 262a is exposed through the opening 261b. When the basket forceps adapter 260 is removed from the drape adapter 230, the user presses the portion of the pressing portion 262a exposed through the opening 261b to slide the pressing portion 262a. Thus, the engagement between the engagement claws 233 of the drape adapter 230 and the engagement claws 262b of the basket forceps adapter 260 is released, and the basket forceps adapter 260 can be removed from the drape adapter 230. The engagement between the engagement claws 233 of the drape adapter 230 and the engagement claws 262b of the basket forceps adapter 260 is similar to the engagement between the engagement claws 226 of the hand 220 and the engagement claws 232b of the drape adapter 230 shown in FIG. 13.

In the second embodiment, as shown in FIG. 24, the basket forceps adapter rotator 263 engages with the drape adapter rotator 235 and rotates together with the drape adapter rotator 235. The basket forceps adapter rotator 263 has a disk shape, for example. The engagement recess 263a that engages with the engagement protrusion 235b of the drape adapter rotator 235 is formed on the back surface of the basket forceps adapter rotator 263 facing the drape adapter 230. As shown in FIG. 25, the basket forceps mover 264 is attached to the front surface of the basket forceps adapter rotator 263 opposite to the drape adapter 230.

As shown in FIG. 25, the basket forceps mover 264 includes a pinion 264a, a rack 264b that meshes with the pinion 264a, and a mover 264c shown in FIG. 23 that is connected to the rack 264b and moves a forceps operation unit 303a. A recess is formed in the mover 264c, and the forceps operation unit 303a is placed in the recess. When the basket forceps adapter rotator 263 rotates, the forceps operation unit 303a moves linearly together with the mover 264c. Thus, a distal end of the basket forceps 303 is opened and closed within the urethra 301 of the patient 300.

As shown in FIG. 23, the holder 265 is disposed on the outer surface of the housing 261 of the basket forceps adapter 260 and holds the basket forceps 303. Specifically, the holder 265 includes a plurality of protrusions that protrude from the outer surface of the housing 261. The basket forceps 303 is disposed between the plurality of protrusions. The holder 265 includes holders 265a that hold the basket forceps 303 between the holders 265a and the mover 264c, and a U-shaped holder 265b.

### Advantageous Effects of Second Embodiment

The robot system 210 includes the endoscope adapter 240 including the endoscope mover 244 and configured to be attached to and detached from the hand 220. Accordingly, the ureteroscope 200 can be easily attached to and detached from the hand 220.

The endoscope adapter 240 includes the endoscope adapter attachment/detachment portion 242 to receive manual operations to attach and detach the endoscope adapter 240 to and from the hand 220. Accordingly, the user can easily attach and detach the endoscope adapter 240 to and from the hand 220 simply by operating the endoscope adapter attachment/detachment portion 242. Therefore, user convenience can be improved when the endoscope adapter 240 is attached to and detached from the hand 220.

The robot system 210 includes the drape adapter 230 to which the drape 310 is attached and configured to be attached to and detached from the hand 220. The endoscope adapter 240 is attached to the hand 220 via the drape adapter 230. Accordingly, the endoscope adapter 240 can be replaced while the hand 220 is covered with the drape 310, and thus attachment of germs and the like to the affected area of the patient 300 can be reduced or prevented.

The drape adapter 230 includes the drape adapter attachment/detachment portion 232 to receive manual operations to attach and detach the drape adapter 230 to and from the hand 220. Accordingly, the user can easily attach and detach the drape adapter 230 to and from the hand 220 simply by operating the drape adapter attachment/detachment portion 232. Therefore, user convenience can be improved when the drape adapter 230 is attached to and detached from the hand 220.

The drape adapter 230 includes the drape adapter rotator 234 configured to be rotated by the driving force of the endoscope drive 222, and the endoscope adapter 240 includes the endoscope adapter rotator 243 configured to engage with the drape adapter rotator 234 and rotate together with the drape adapter rotator 234. The endoscope mover 244 is connected to the endoscope adapter rotator 243 and is configured to rotate together with rotation of the endoscope adapter rotator 243. Accordingly, the driving force of the endoscope drive 222 can be easily transmitted to the endoscope mover 244 via the drape adapter rotator 234 and the endoscope adapter rotator 243.

The robot system 210 includes the laser fiber adapter 250 including the laser fiber mover 254 and configured to be manually attached to and detached from the hand 220, and the basket forceps adapter 260 including the basket forceps mover 264 and configured to be manually attached to and detached from the hand 220. Accordingly, the user can easily replace the laser fiber adapter 250 and the basket forceps adapter 260, and thus user convenience can be improved when the laser fiber adapter 250 and the basket forceps adapter 260 are replaced.

The robot system 210 includes the laser fiber adapter 250 for the laser fiber 304 configured to fragment stones by laser light irradiation, and the basket forceps adapter 260 for the basket forceps 303 configured to retrieve the stones. Accordingly, the robot system 210 can perform a procedure on the patient 300 using both the laser fiber 304 and the basket forceps 303.

The robot system 210 includes the drape adapter 230 to which the drape 310 is attached and configured to be attached to and detached from the hand 220. Either the laser fiber adapter 250 or the basket forceps adapter 260 is attached to the hand 220 via the drape adapter 230. Accordingly, the laser fiber adapter 250 and the basket forceps adapter 260 can be exchanged with each other while the hand 220 is covered with the drape 310, and thus attachment of germs and the like to the affected area of the patient 300 can be reduced or prevented.

### Modified Examples

The embodiments disclosed this time must be considered as illustrative in all points and not restrictive. The scope of the present disclosure is not shown by the above description of the embodiments but by the scope of claims for patent, and all modifications (modified examples) within the meaning and scope equivalent to the scope of claims for patent are further included.

For example, while the example in which the robot arm 12 is a vertical articulated robot arm 12 has been shown in each of the aforementioned first and second embodiments, the present disclosure is not limited to this. For example, the robot arm 12 may be a horizontal articulated robot arm 12.

While the example in which the operation unit 20 includes the joysticks 21 and 22, the directional pad 23, and the plurality of buttons 24 has been shown in each of the aforementioned first and second embodiments, the present disclosure is not limited to this. For example, the operation unit 20 may include a touch panel or the like.

While the example in which the controller 30 is a robot controller has been shown in each of the aforementioned first and second embodiments, the present disclosure is not limited to this. For example, the controller 30 may be a PC (personal computer) that is disposed separately from the robot controller.

While the example in which the ureteroscope 200 is applied as a treatment instrument according to the present disclosure has been shown in each of the aforementioned first and second embodiments, the present disclosure is not limited to this. For example, an endoscope inserted into the respiratory organs such as the lungs and bronchi, or the digestive organs such as the stomach and large intestine may be applied as the treatment instrument according to the present disclosure.

While the example in which the robot 10 operates to retrace the movement path M along which the ureteroscope 200 is inserted into the patient 300 when the ureteroscope 200 is removed from the patient 300 has been shown in each of the aforementioned first and second embodiments, the present disclosure is not limited to this. For example, when the ureteroscope 200 is reinserted into the patient 300, the controller 30 may control the operation of the robot 10 to retrace the movement path M of the ureteroscope 200 based on the information about the movement path M of the ureteroscope 200 stored in the storage 40. That is, when the ureteroscope 200 is reinserted into the patient 300, the controller 30 may operate the robot 10 to retrace the movement path M along which the ureteroscope 200 was previously inserted into the patient 300. For example, when the ureteroscope 200 is reinserted into the patient 300, the button 24b of the plurality of buttons 24 disposed on the operation unit 20 is pressed such that the robot 10 operates to retrace the movement path M along which the ureteroscope 200 was previously inserted into the patient 300. Thus, when the ureteroscope 200 is reinserted into the patient 300, the robot 10 automatically operates to retrace the movement path M of the ureteroscope 200, and thus the burden on the user can be reduced when the ureteroscope 200 is reinserted into the patient 300.

While the example in which when the button 24a of the operation unit 20 is pressed, the robot 10 operates such that the ureteroscope 200 retraces the movement path M stored in the storage 40 toward the A2 side has been shown in each of the aforementioned first and second embodiments, the present disclosure is not limited to this. For example, when the joystick 21 receives an operation to move the ureteroscope 200 in the backward direction A2, the controller 30 may control the operation of the robot 10 to retrace the movement path M of the ureteroscope 200 based on the information about the movement path M of the ureteroscope 200 stored in the storage 40. That is, when the ureteroscope 200 is moved backward, the controller 30 operates the robot 10 to retrace the movement path M, which is stored in the storage 40 and corresponds to the movement path M along which the ureteroscope 200 is moved forward. Thus, when the ureteroscope 200 is moved in the backward direction within the body of the patient 300, the robot 10 automatically operates to constantly retrace the movement path M of the ureteroscope 200. Consequently, the burden on the user can be reliably reduced when the ureteroscope 200 is moved in the backward direction.

While the example in which when the button 24a of the operation unit 20 is pressed, the robot 10 operates such that the ureteroscope 200 retraces the movement path M stored in the storage 40 toward the A2 side has been shown in each of the aforementioned first and second embodiments, the present disclosure is not limited to this. For example, when the button 24 other than the button 24a of the operation unit 20 is operated, the robot 10 may operate such that the ureteroscope 200 retraces the movement path M stored in the storage 40 toward the A2 side.

While the example in which the robot 10 retraces the movement path M of the ureteroscope 200 while the button 24a of the operation unit 20 is continuously pressed has been shown in each of the aforementioned first and second embodiments, the present disclosure is not limited to this. For example, when the button 24a of the operation unit 20 is pressed once, the operation of the robot 10 to retrace the movement path M of the ureteroscope 200 may be started, and the operation of the robot 10 to retrace the movement path M of the ureteroscope 200 may continue until the button 24a of the operation unit 20 is pressed a second time.

While the example in which the entire overlapping portion of the information about the movement path M is deleted from the storage 40 has been shown in each of the aforementioned first and second embodiments, the present disclosure is not limited to this. For example, only a portion of the overlapping portion of the information about the movement path M may be deleted from the storage 40. Alternatively, the overlapping portion of the information about the movement path M may not be deleted from the storage 40. Thus, the robot 10 operates to retrace the entire movement path traveled by the ureteroscope 200 within the body of the patient 300.

While the example in which the robot 10 includes the operation shaft JT7 to bend the ureteroscope 200 has been shown in each of the aforementioned first and second embodiments, the present disclosure is not limited to this. For example, the present disclosure can also be applied to a robot 10 that does not include the operation shaft JT7.

The functionality of the elements disclosed herein may be implemented using circuitry or processing circuitry which includes general purpose processors, special purpose processors, integrated circuits, ASICs ("Application Specific Integrated Circuits"), conventional circuitry and/or combinations thereof which are configured or programmed to perform the disclosed functionality. Processors are considered processing circuitry or circuitry as they include transistors and other circuitry therein. The processor may be a programmed processor which executes a program stored in a memory. In the disclosure, the circuitry, units, or means are hardware that carry out or are programmed to perform the recited functionality. The hardware may be any hardware disclosed herein or otherwise known which is programmed or configured to carry out the recited functionality. When the hardware is a processor which may be considered a type of circuitry, the circuitry, means, or units are a combination of hardware and software, the software being used to configure the hardware and/or processor.

### Aspects

It will be appreciated by those skilled in the art that the exemplary embodiments described above are specific examples of the following aspects.

### (Item 1)

A robot system comprising:
a robot to hold a treatment instrument to be inserted into a patient;
an operation unit to receive an operation for the robot;
a controller configured or programmed to control operation of the robot based on the operation received by the operation unit; and
a storage; wherein
the controller is configured or programmed to:
   store, in the storage, information about a movement path of the treatment instrument configured to pass through a body of the patient; and
   control the operation of the robot to retrace the movement path of the treatment instrument based on the information about the movement path of the treatment instrument stored in the storage.

### (Item 2)

The robot system according to item 1, wherein the controller is configured or programmed to:
store, in the storage, the information about the movement path of the treatment instrument along which the treatment instrument is inserted into the patient; and
control the operation of the robot to retrace the movement path of the treatment instrument along which the treatment instrument is inserted based on the information about the treatment instrument stored in the storage when the treatment instrument is removed from the patient.

### (Item 3)

The robot system according to item 1 or 2, wherein the controller is configured or programmed to, when the treatment instrument is reinserted into the patient, control the operation of the robot to retrace the movement path of the treatment instrument based on the information about the movement path of the treatment instrument stored in the storage.

### (Item 4)

The robot system according to any one of items 1 to 3, wherein
the treatment instrument includes a ureteroscope to be inserted into a urethra of the patient; and
the operation unit is configured to receive operations to rotate and bend the ureteroscope and to move the ureteroscope in a forward-backward direction.

### (Item 5)

The robot system according to any one of items 1 to 4, wherein the controller is configured or programmed to:
store, in the storage, at least one of a position of the treatment instrument in a forward-backward direction, a rotation angle of the treatment instrument, or a bending angle of the treatment instrument at predetermined time intervals or at predetermined movement distance intervals as the information about the movement path of the treatment instrument; and
control the operation of the robot to retrace the movement path of the treatment instrument based on at least one of the position of the treatment instrument in the forward-backward direction, the rotation angle of the treatment instrument, or the bending angle of the treatment instrument stored in the storage.

### (Item 6)

The robot system according to any one of items 1 to 5, wherein the operation unit includes a first operation unit to receive an operation to start the operation of the robot to retrace the movement path.

### (Item 7)

The robot system according to item 6, wherein the robot is configured to retrace the movement path of the treatment instrument while the first operation unit is continuously operated.

### (Item 8)

The robot system according to any one of items 1 to 7, wherein
the operation unit includes a second operation unit to receive an operation to move the treatment instrument in a forward-backward direction; and
the controller is configured or programmed to, when the second operation unit receives an operation to move the treatment instrument in a backward direction, control the operation of the robot to retrace the movement path of the treatment instrument based on the information about the movement path of the treatment instrument stored in the storage.

### (Item 9)

The robot system according to any one of items 1 to 8, wherein the controller is configured or programmed to control the operation of the robot such that the treatment instrument moves at a predetermined speed or less when the movement path of the treatment instrument is retraced.

### (Item 10)

The robot system according to any one of items 1 to 9, wherein the controller is configured or programmed to execute a process to delete, from the storage, at least a portion of an overlapping portion of the information about the movement path previously stored in the storage when the treatment instrument moves back and forth within the body of the patient and forward and backward paths of the treatment instrument overlap.

### (Item 11)

The robot system according to any one of items 1 to 10, wherein the operation unit is configured to receive operations to move the treatment instrument up and down.

### (Item 12)

The robot system according to any one of items 1 to 11, wherein
the robot includes a robot arm to hold the treatment instrument; and
the controller is configured or programmed to control operation of the robot arm and operation of an operation shaft configured to operate the treatment instrument so as to retrace the movement path of the treatment instrument based on the information about the movement path of the treatment instrument stored in the storage.

### (Item 13)

A method for controlling a robot system, the method comprising:
storing, in a storage, information about a movement path along which a treatment instrument held by a robot passes through a body of a patient as the robot operates based on an operation received by an operation unit; and
controlling operation of the robot to retrace the movement path of the treatment instrument based on the information about the movement path of the treatment instrument stored in the storage.

## Claims

1. A robot system comprising:
a robot to hold a treatment instrument to be inserted into a patient;
an operation unit to receive an operation for the robot;
a controller configured or programmed to control operation of the robot based on the operation received by the operation unit; and
a storage; wherein
the controller is configured or programmed to:
store, in the storage, information about a movement path of the treatment instrument configured to pass through a body of the patient; and
control the operation of the robot to retrace the movement path of the treatment instrument based on the information about the movement path of the treatment instrument stored in the storage.

2. The robot system according to claim 1, wherein the controller is configured or programmed to:
store, in the storage, the information about the movement path of the treatment instrument along which the treatment instrument is inserted into the patient; and
control the operation of the robot to retrace the movement path of the treatment instrument along which the treatment instrument is inserted based on the information about the treatment instrument stored in the storage when the treatment instrument is removed from the patient.

3. The robot system according to claim 1, wherein the controller is configured or programmed to, when the treatment instrument is reinserted into the patient, control the operation of the robot to retrace the movement path of the treatment instrument based on the information about the movement path of the treatment instrument stored in the storage.

4. The robot system according to claim 1, wherein
the treatment instrument includes a ureteroscope to be inserted into a urethra of the patient; and
the operation unit is configured to receive operations to rotate and bend the ureteroscope and to move the ureteroscope in a forward-backward direction.

5. The robot system according to claim 1, wherein the controller is configured or programmed to:
store, in the storage, at least one of a position of the treatment instrument in a forward-backward direction, a rotation angle of the treatment instrument, or a bending angle of the treatment instrument at predetermined time intervals or at predetermined movement distance intervals as the information about the movement path of the treatment instrument; and
control the operation of the robot to retrace the movement path of the treatment instrument based on at least one of the position of the treatment instrument in the forward-backward direction, the rotation angle of the treatment instrument, or the bending angle of the treatment instrument stored in the storage.

6. The robot system according to claim 1,
wherein the operation unit includes a first operation unit to receive an operation to start the operation of the robot to retrace the movement path.

7. The robot system according to claim 6,
wherein the robot is configured to retrace the movement path of the treatment instrument while the first operation unit is continuously operated.

8. The robot system according to claim 1, wherein
the operation unit includes a second operation unit to receive an operation to move the treatment instrument in a forward-backward direction; and
the controller is configured or programmed to, when the second operation unit receives an operation to move the treatment instrument in a backward direction, control the operation of the robot to retrace the movement path of the treatment instrument based on the information about the movement path of the treatment instrument stored in the storage.

9. The robot system according to claim 1, wherein the controller is configured or programmed to control the operation of the robot such that the treatment instrument moves at a predetermined speed or less when the movement path of the treatment instrument is retraced.

10. The robot system according to claim 1, wherein the controller is configured or programmed to execute a process to delete, from the storage, at least a portion of an overlapping portion of the information about the movement path previously stored in the storage when the treatment instrument moves back and forth within the body of the patient and forward and backward paths of the treatment instrument overlap.

11. The robot system according to claim 1, wherein the operation unit is configured to receive operations to move the treatment instrument up and down.

12. The robot system according to claim 1, wherein
the robot includes a robot arm to hold the treatment instrument; and
the controller is configured or programmed to control operation of the robot arm and operation of an operation shaft configured to operate the treatment instrument so as to retrace the movement path of the treatment instrument based on the information about the movement path of the treatment instrument stored in the storage.

13. A method for controlling a robot system, the method comprising:
storing, in a storage, information about a movement path along which a treatment instrument held by a robot passes through a body of a patient as the robot operates based on an operation received by an operation unit; and
controlling operation of the robot to retrace the movement path of the treatment instrument based on the information about the movement path of the treatment instrument stored in the storage.
